# EUROPEAN PATENT APPLICATION

(11) **EP 1 484 193 A2**
(43) Date of publication of application: **08.12.2004**
(21) Application number: 04253193.9
(22) Date of filing: 28.05.2004
(51) Int. Cl.: B42F 7/06, A61J 1/00

(54) **Improvements in and relating to treatment programme packs**

(30) Priority: 03.06.2003 GB 0312692
(71) Applicant: Bringhigh Limited, Broughton, Nr Chester, Clwyd CH4 0EG (GB)
(72) Inventor: Kitchen, John Tanfield, Bringhigh Limited, Nr Chester, Clwyd, CH4 0EG (GB); Jones, John Harold, Bringhigh Limited, Nr Chester Clwyd, CH4 0EG (GB)
(74) Representative: Lyons, Andrew John

(57) **Abstract**

A treatment programme pack (10) comprising a folio having first (12) and second leaves (14), the first (12) and second (14) leaves being hingedly connected to one another, wherein the first leaf (12) is adapted to receive a medication pack (26) and the second leaf (14) is adapted to receive documents (30). The pack (10) may comprise provision for retaining a patient monitor (46) instead of/ in addition to the documents (30). A blank (52) for forming the pack (10) is also disclosed.

## Description

The present invention concerns improvements in and relating to treatment programme packs.

In particular, the present invention relates to improvements in and relating to treatment programme packs for retaining medication packs.

When a patient is discharged from hospital or when they are undergoing long-term care or rehabilitation, they are often subjected to a fairly strict and/or complicated medication regime. A particular medication regime may require the patient to take different medications at different times of day, for a relatively long period of time, say from anything between a week to indefinitely. Furthermore, the patient may require therapy, repeat visits to doctors or other healthcare professionals, and/or may need to monitor him or herself.

Accordingly, when some patients are discharged from hospital, or admitted long-term, they are required to remember and implement an often complex treatment programme. They may be given instructions from a number of healthcare professionals and numerous appointments for repeat visits or outpatient clinics. This information is sometimes difficult to comprehend or assimilate, and is often lost, giving rise to time wastage and consequential economic loss. More importantly, however, if the patient fails to keep to a treatment programme, their health may deteriorate irreversibly.

Patients generally like to be in control of their own treatment programme, since it reduces their reliance on healthcare professionals, thereby promoting the healing process or reducing feelings of helplessness, stress and anxiety etc.
However, certain patients are not capable or find it very difficult to self-administer medication or to remember appointments.

There are a number of products on the market aimed at improving patient compliance, especially with respect to medication regimes, for example, the Venalink home medication pack system (Venalink is a registered trade mark), for which a UK Patent has been applied for (GB0312224.9) that provides a plurality of blisters for retaining medication doses corresponding to different times of the day. The Venalink system is prepared by a healthcare professional, such as a pharmacist, to ensure that the correct medication doses are taken at the correct times.

Although systems, such as the Venalink system are useful, they do not provide a holistic solution to the problem of encouraging patients to keep to a treatment regime inclusive of repeat visits to healthcare professionals and other activities that may encourage healing or reduce discomfort.

The present invention aims to overcome one or more of the above problems by providing an improved treatment programme pack.

Accordingly, a first aspect of the present invention provides a treatment programme pack comprising a folio having first and second leaves, the first and second leaves being hingedly connected to one another, wherein the first leaf is adapted to receive a medication pack and the second leaf is adapted to receive documents.

Preferably, the treatment programme pack is manufactured of a durable material, such as a plastics material, although laminated card could also be used. The material from which the pack is manufactured is preferably transparent or translucent to allow the medication pack and/or documents to be more easily identified without having to remove them from the pack.

The hinged connection of the first and second leaves is preferably by way of a pre-formed crease, or line of weakness formed in the material of the pack. There is also, preferably, a spine positioned between the leaves of the folio so that the leaves are spaced apart when in a closed position.

Where the first leaf is adapted to receive a medication pack, it is preferably adapted to do so by the provision of a pocket therein or thereon. Folding and gluing the leaf along all but one edge to form a pocket may integrally form the pocket with the leaf, for example, or it may be formed separately and affixed thereto.

Additionally or alternatively, the glued joints described above, may be welded.

Alternative means may also be used to form a pocket, either integrally with or separately form the first leaf for example, by the provision of tabs that engage with a separate sheet or by affixing a pre-formed pocket to the first leaf respectively.

Where the second leaf of the medication pack is adapted to receive documents, this may also be by way of the provision of one or more pockets therein or thereon. Where more than one pocket is provided, they may be integrally formed with each other by the providing a slit in the first pocket that enables further documents to be inserted therein. The slits, where provided are preferably adapted for receiving smaller documents than the main pocket.

A second aspect of the present invention provides a treatment programme pack comprising folio having a two or more leaves, the leaves being hingedly connected to one another and adapted to receive documents, wherein the treatment programme pack further comprises means for retaining a medication pack.

The treatment programme pack may be adapted to retain a medication pack by having one or more formations that engage with the medication pack. The formations are preferably press-studs that engage with apertures formed in the medication pack.

In a preferred embodiment of the invention, the formations are mounted on a flap. The flap may be hingedly connected to the treatment programme pack. The flap may be formed integrally with the treatment programme pack or it may be affixed to thereto. Additionally or alternatively, the flap may be detachable.

In a most preferred embodiment, the medication pack is retained on the treatment programme pack by a two-leaf flap that is hingedly connected thereto. The two leaf flap preferably comprises first and second leaves having male and female press studs thereon respectively. The press-studs are most preferably arranged to coincide with apertures formed in the medication pack. According to this preferred embodiment of the invention, the medication pack can therefore be lifted or folded back to enable easier access to the compartments of the treatment programme pack.

A third aspect of the present invention provides a system for assisting a patient in complying with a treatment programme comprising a folio for retaining a medication pack, information regarding appointments, personal details and self-administrable therapy, wherein the folio is adapted for receiving a patient monitor.

The patient monitor is preferably retained by the treatment programme pack by the provision of a board having formations therein for engaging with the patient monitor. In a preferred embodiment of the invention, the treatment programme pack is retained on a foam board provided with cut outs, such that the patient monitor is a push fit therein. Additionally or alternatively, straps, additional compartments may be provided for retaining the patient monitor.

The patient monitor may be of any type. However, it is envisaged that monitors that can be easily operated by the patient at home, would be most likely supplied with the system. Suitable patient monitors may therefore be, amongst others, blood oximiters, blood sugar monitors, thermometers and /or cardio-vascular monitors.

A fourth aspect of the present invention provides a blank for assembling into a treatment programme pack folio comprising;
first, second, third, fourth and fifth adjacent leaves; and
first, second, third and fourth tabs for affixing to either the first or fifth leaf; wherein
the first and second tabs are adjoined to the second leaf; and the third and fourth tabs are adjoined to the fourth leaf; and wherein
the third leaf is narrower than the first, second, fourth and fifth leaves for forming a spine when the blank is assembled.

A preferred embodiment of the invention shall now be described, by way of example only, with reference to the accompanying drawings, in which;
Figure 1 shows a perspective view of a preferred embodiment of the first aspect of the invention;
Figure 2 shows a perspective view of a preferred embodiment of the first aspect of the invention in use;
Figure 3 shows a perspective view of a preferred embodiment of the second aspect of the invention;
Figure 4 shows a perspective view of a preferred embodiment of the third aspect of the invention;
Figure 5 shows a blank according to the fourth aspect of the invention;
Figure 6 shows a blank of the flap used for retaining a medication pack; and
Figure 7 shows the engagement of a medication pack with the treatment programme pack of the invention using add-on flaps.

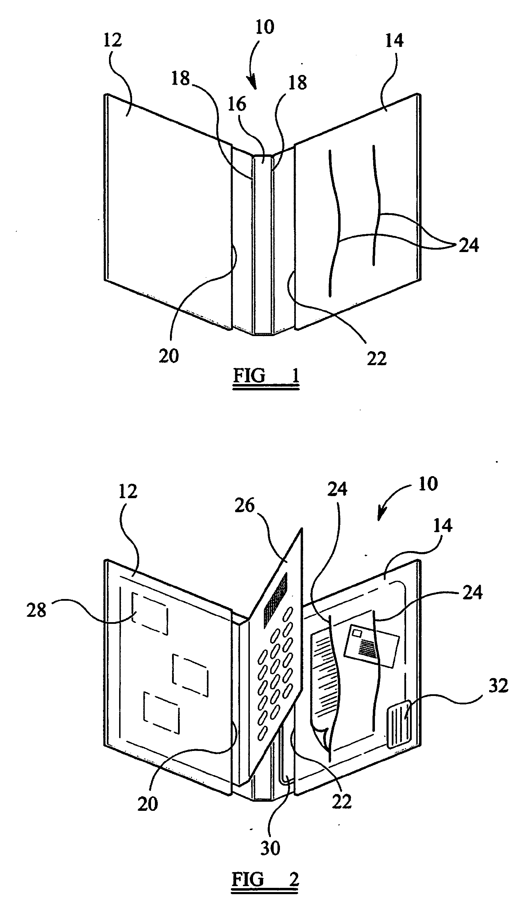

Figure 1 shows a treatment programme pack 10 having first 12 and second 14 leaves hingedly connected to one another.

A spine 16 is also provided, such that the first 12 and second 14 leaved are spaced apart when folded towards each other. Lines of weakness 18 are provided to facilitate folding the 14 treatment programme pack 10. The first leaf 12 has a pocket 20 thereon for retaining a medication pack (not shown). The second leaf 14, also has a pocket 22 for retaining documents (not shown). The pocket 22 of the second leaf also has slits 24 for retaining/dividing the documents (not shown).

Figure 2 shows a treatment programme pack 10 as per Figure 1 in use. The pocket 20 of the first leaf 12 is used fore retaining a medication pack 26. The treatment programme pack 10 is manufactured of a translucent material so that the details 28 of the medication pack 26 can be viewed through the pocket 20 of the first leaf 12 without having to be removed.

The pocket 22 of the second leaf 14 is used for retaining documents 30. The slits 24 are used for sorting the documents say by size or type. The slot 24' is larger than slot 24" in the illustrated embodiment. The preferred slit is curved, preferably in the manner of a shallow U-shape to aid insertion and removal of documents.

Again, because the treatment programme pack 10 is manufactured of a translucent material, the documents can be more easily identified without being removed. There is also provided an area 32 for identifying the patient to whom the pack pertains by way of name, code or reference or symbol.

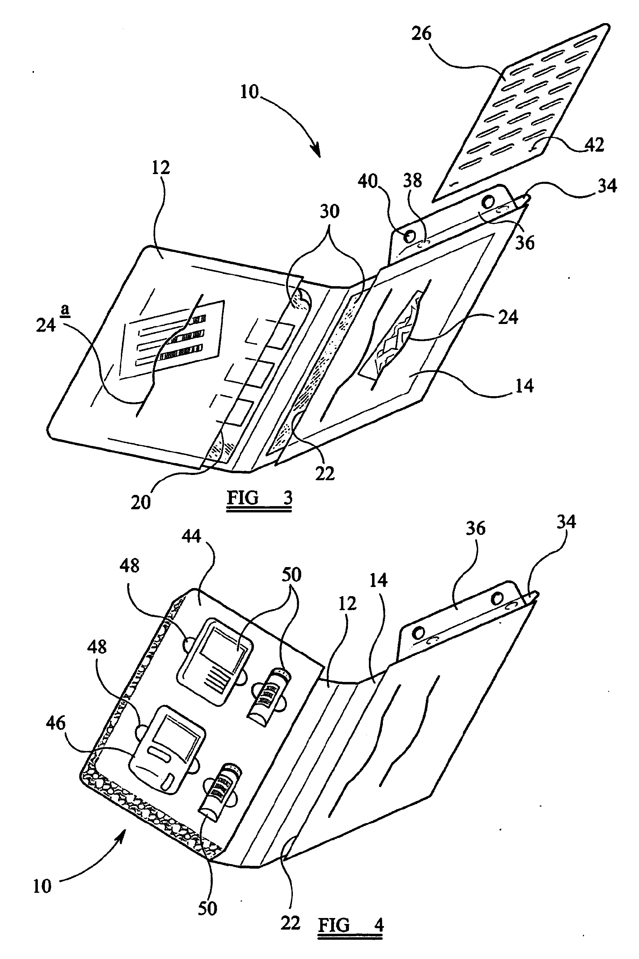

Turning now to Figure 3 of the drawings, a treatment programme pack 10 according to the second aspect of the invention is shown. In this embodiment, the pockets 20 & 22 of both leaves 12 & 14 are used for retaining documents 30. A further slit 24a is provided in the pocket 20 of the first leaf 12 for convenience.

Flaps 34 & 36 are provided for retaining a medication pack 26. One of the flaps 34 has a male press-stud 38 thereon and the other flap 36 has a female press-stud 40 thereon. The press-studs 38 & 40 are arranged to engage with apertures 42 of the medication pack 26. The medication pack 26 can therefore be retained by the flaps 34 & 36 whilst being relatively free to be folded up for easier access to the pockets 20 & 22 of the treatment programme pack 10.

Figure 4 shows the treatment programme pack 10 according to the third aspect of the invention whereby the pocket on the first leaf 12 is replaced by a patient monitor retaining board 44. The monitor retaining board 44 is manufactured of a foam material and has cutouts for conveniently retaining a patient monitor 46. The cutouts have finger holes 48 to facilitate extraction of the patient monitor from the board 44. The board 44 is also adapted to receive other items 50 such as needles for a blood monitor, swabs, batteries etc.

The pocket 22 of the second leaf 14 may be adapted for receiving a medication pack (not shown) or flaps 34 & 36 may be provided for that purpose.

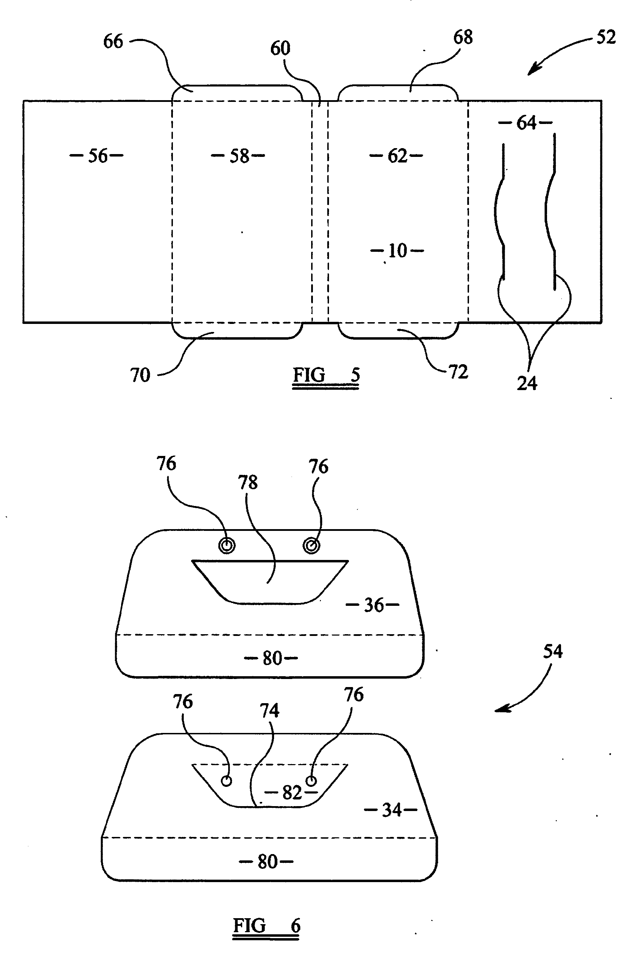

Figures 5 and 6 of the accompanying drawings show blanks 52 & 54 for the treatment programme pack and flaps 34 & 36 respectively.

Figure 5 shows a blank 52 having first 56, second 58, third 60, fourth 62 and fifth 64 leaves and tabs 66, 68, 70 & 72. Folds are indicated by dotted lines. To assemble the treatment programme pack of the invention, the first leaf 56 is folded over the second leaf 58 and the tabs 66 & 70 are used for securing the first leaf 56 in-situ. Likewise, the fifth leaf 64 is folded over the fourth leaf 62 and tabs 68 & 72 are used to secure it in situ. The third leaf 60 forms the spine of the treatment programme pack. The tabs are preferably secured using an adhesive or by welding.

Figure 6 shows blanks for the flaps 34 & 36. The first flap 34, has a slit 74 and apertures 76 therein. The second flap 36 is similar, but instead of a slit, has a relatively large aperture 78 therein. The apertures 76 are used for affixing male and female parts of press-studs (not shown). In use, the flaps 34 & 36 are affixed to the main body of the treatment programme pack by gluing or welding on the tab 80. The tab 82 defined by the slit 74 and fold of the first flap 34 folds through the aperture 78 of the second flap 36. The respective parts of the press-studs then engage with the apertures of the medication pack.

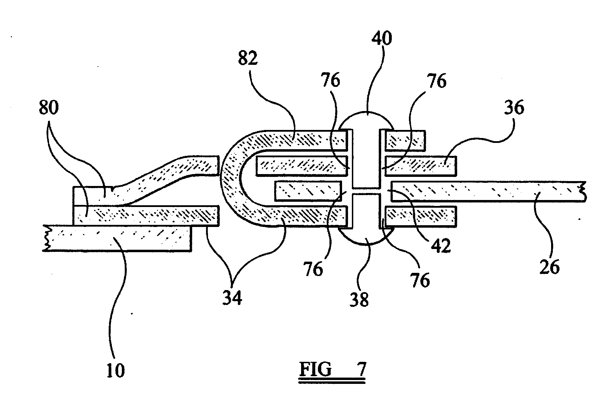

Finally, Figure 7 shows the arrangement of flaps 34 & 36, press-studs 38 & 40, treatment programme pack 10 in use.

## Claims

1. A treatment programme pack (10) comprising a folio having first (12) and second leaves (14), the first (12) and second (14) leaves being hingedly connected to one another, **characterised in that** the first leaf (12) is adapted to receive a medication pack (26) and the second leaf (14) is adapted to receive documents (30).

2. A treatment programme pack (10) as claimed in claim 1, manufactured of a durable material.

3. A treatment programme (10) pack as claimed in claim 2, wherein the durable material is a plastics material or a laminated card material.

4. A treatment programme pack (10) as claimed in claim 1, 2 or 3 wherein the material from which the pack is manufactured is transparent or translucent.

5. A treatment programme pack (10) as claimed in any of claims 1 to 4, wherein the hinged connection of the first and second leaves comprises a pre-formed crease or line of weakness (18) formed in the material of the pack.

6. A treatment programme pack (10) as claimed in any of claims 1 to 5, wherein a spine (16) is positioned between the leaves (12 & 14) of the folio so that the leaves are spaced apart when in a closed position.

7. A treatment programme pack (10) as claimed in any of claims 1 to 6, wherein the first leaf (12) is adapted to receive a medication pack (26) by the provision of a pocket (20) therein or thereon.

8. A treatment programme pack (10) as claimed in any of claims 1 to 7, wherein one or more joints of the medication pack are welded.

9. A treatment programme pack (10) as claimed in any of claims 1 to 8, wherein the second leaf (14) of the medication pack is adapted to receive documents.

10. A treatment programme pack (10) as claimed in any of claims 1 to 9, wherein the second leaf (14) has one or more pockets (22) therein or thereon.

11. A treatment programme pack (10) as claimed in claim 10, wherein where more than one pocket (22) is provided, they are integrally formed with each other by the provision of a slit (24) in the first pocket (22) that enables further documents (30) to be inserted therein.

12. A treatment programme pack (10) as claimed in claim 11, wherein the slits (24), are adapted for receiving smaller documents than the main pocket.

13. A treatment programme pack (10) comprising a folio having two or more leaves (12 & 14), the leaves (12 &14) being hingedly connected to one another and adapted to receive documents (30), wherein the treatment programme pack further comprises means for retaining a medication pack (26).

14. A treatment programme pack (10) as claimed in claim 13, adapted to retain a medication pack (26) by having one or more formations (40) that engage with the medication pack.

15. A treatment programme pack (10) as claimed in claim 14, wherein the formations are press-studs (40) that engage with apertures (42) formed in the medication pack (26).

16. A treatment programme pack (10) as claimed in claim 14 or claim 15, wherein the formations (40) are mounted on a flap (36) formed integrally or detachable from the treatment programme pack.

17. A treatment programme pack (10) as claimed in any of claims 13 to 16, wherein the medication pack (26) is retained on the treatment programme pack (10) by a two-leaf flap (26) that is hingedly connected thereto.

18. A treatment programme pack (10) as claimed in claim 17, wherein the two-leaf flap (36) comprises first (34) and second leaves (36) having male and female press-studs (40) thereon.

19. A treatment programme pack (10) as claimed in claim 18, wherein the press-studs (40) are arranged to coincide with apertures (42) formed in the medication pack.

20. A system (10) for assisting a patient in complying with a treatment programme comprising a folio for retaining a medication pack (26), information regarding appointments, personal details and self-administrable therapy (28), wherein the folio is adapted for receiving a patient monitor (46).

21. A system (10) as claimed in claim 20, wherein the patient monitor (46) is retained by the treatment programme pack by the provision of a board (44) having formations (48) therein for engaging with the patient monitor.

22. A blank (53 & 54) for assembling into a treatment programme pack folio comprising;
first (56), second (58), third (60), fourth (62) and fifth (64) adjacent leaves; and
first second, third and fourth tabs (66, 68, 70 & 72) for affixing to either the first (56) or fifth leaf (64); wherein
the first (66) and second tabs (68) are adjoined to the second leaf (58); and the third (70) and fourth tabs (72) are adjoined to the fourth leaf (62); and wherein
the third leaf (60) is narrower than the first (56), second (58), fourth (62) and fifth (64) leaves for forming a spine (16) when the blank (53) is assembled.
